# EUROPEAN PATENT APPLICATION

(11) **EP 4 487 806 A1**
(43) Date of publication of application: **08.01.2025**
(21) Application number: 23315273.5
(22) Date of filing: 07.07.2023
(51) Int. Cl.: A61B 34/30, A61F 2/24, A61B 17/00

(54) **CARDIOVASCULAR NAVIGATION AND ACTUATION SYSTEM, CARDIOVASCULAR SYSTEM COMPRISING A CARDIOVASCULAR NAVIGATION AND ACTUATION SYSTEM, MECHANICAL INTERFACE, KIT OF PARTS**

(71) Applicant: Caranx Medical, 75013 Paris (FR)
(72) Inventor: SCHEGG, Pierre, 06000 Nice (FR); SMITS, Jonas Victor Harmen, 3360 Bierbeek (BE); CERRUTI, Giulio, 06700 Saint-Laurent-Du-Var (FR); BERTHET-RAYNE, Pierre, 306800 Cagnes-sur-Mer (FR)
(74) Representative: Hepp Wenger Ryffel AG

(57) **Abstract**

A cardiovascular navigation and actuation system (1) is disclosed. It comprises at least one of and preferably both of a distal unit (2) for translating and/or rotating a cardiovascular delivery system (3), in particular a TAVI or a TAVR delivery system, along its longitudinal axis; and a proximal unit (4) comprising an actuation module (6) for actuating a function of a handle (7) of the delivery system (3), in particular to deploy an artificial cardiovascular valve (40), wherein the mechanical interface (5) provides a connection between the handle (7) and the actuation module (6). In addition, a cardiovascular system is disclosed which comprises a cardiovascular navigation and actuation system (1), a positioning unit and a control unit for controlling the cardiovascular navigation and actuation system (1) and the positioning unit. A mechanical interface, a kit of parts, and a method of using a cardiovascular navigation and actuation system (1) are also disclosed.

## Description

The present invention pertains to cardiovascular navigation and actuation systems, to cardiovascular systems comprising a cardiovascular navigation and actuation system, to mechanical interfaces, to kits of parts, and to methods of using a cardiovascular navigation and actuation system.

Such systems and methods may be used, for example, for navigating a TAVR (Transcatheter Aortic Valve Replacement) or a TAVI (Transcatheter Aortic Valve Implantation) delivery system (i.e., to move its tip where a prosthetic valve is loaded from a percutaneous access site to a native aortic valve across the vascular network) and to actuate the handle of such a system to deploy a valve in a target location and configuration.

US 2021/0236773 A1 discloses a robotic system comprising a robotic catheter steerable by a motorized drive system.

Shaikh et al. (The Amigo Remote Catheter System: From Concept to Bedside. J Innov Card Rhythm Manag. 2017 Aug 15;8(8):2795-2802, doi: 10.19102/icrm.2017.080806. PMID: 32494463; PMCID: PMC7252924, https://www.innovationsincrm.com/images/pdf/crm-08-08-2795.pdf) discloses a remote catheter system using a catheter and handle designed for the system.

Handles of delivery systems are very different depending on the manufacturer. Automated systems disclosed in the prior art have the disadvantage that they can cooperate with only one single kind of handle which has been specifically designed for use with this system, and none of the commercially available hand-held systems.

It is one object of the present invention to overcome the disadvantages in the prior art. In particular, a cardiovascular system should be provided which allows to employ different delivery systems.

In one aspect, the invention pertains to a cardiovascular navigation and actuation system comprising at least one of and preferably both of
- a distal unit for translating and/or rotating a cardiovascular delivery system along its longitudinal axis;
- a proximal unit comprising an actuating module for actuating a function of a handle of the delivery system, in particular to deploy an artificial cardiovascular valve.

The actuating module may be designed for at least one of:
- actuating a handle of the delivery system for deploying a self-expanding artificial valve;
- actuating a handle of a delivery system for deploying a balloon-expanding artificial valve;
- moving concentric catheters relative to one another, in particular inside a patient's body;
- flexing a catheter body, in particular a tip of the catheter body;
- rotating a balloon catheter;
- engaging and/or disengaging a balloon lock;
- moving a concentric guidewire relative to a delivery system or catheter.

In some embodiments, actuation of any of the functions of the handle may be achieved by human gesture recognition.

Advantageously, the proximal unit comprises a mechanical interface which provides a connection between the handle and the actuation module. The mechanical interface can transfer forces between the actuation module and the handle.

The cardiovascular navigation and actuation system may further contain the delivery system or a portion of it, e.g., a delivery catheter.

A cardiovascular navigation and actuation system according to the invention has the advantage that only the proximal unit, in particular only the mechanical interface, has to be specifically adapted to cooperate with a specific delivery system handle. The mechanical interface can be an integral part of the actuation module. It can also be formed by one or more adaptors which can be brought in operative connection with the actuation module.

Preferably, the proximal unit is also configured to load and/or unload the delivery system. It may therefore serve several purposes.

In one embodiment, the distal unit may be adapted to be arranged near a percutaneous access site. It can, for example, be clamped directly on the patient, on the side of a bed, or on an active manipulator, such as a robot, or on a passive manipulator. The passive manipulator may be a positioning arm which may, in particular, be telescopic.

With preference, the actuation module is mounted on a proximal positioning unit, in particular an active positioning unit such as a robot, or a passive positioning unit. It may be designed and arranged to move along a cardiovascular instrument, to move the cardiovascular instrument, to anchor the cardiovascular instrument to the handle, or to deploy the artificial valve.

Also with preference, the distal unit is mounted on a distal positioning unit, in particular an active positioning unit such as a robot, or a passive positioning unit.

The proximal unit and the distal unit may be arranged to move independently from one another. These movements can be obtained using dedicated positioning units such as robots. Alternatively, there may be passive positioning units or human operators.

In some embodiments, the system further comprises a common frame in which the distal unit and the proximal unit are mounted. This allows to move the distal unit and the proximal unit simultaneously, for example by a robot.

Advantageously, the proximal unit is movable with respect to the distal unit and/or vice versa. This allows an independent motion of these units and increases the range of applicability.

The distal unit may be fixed at a distal end of the common frame. This facilitates the positioning of the distal unit in the vicinity of the percutaneous access site.

When it is aligned, the distal unit may be interfaced with an introducer sheath at the access site and may introduce and advance the delivery system.

The proximal unit may be configured to be moveable towards the distal unit and to enable actuation of the delivery system, e.g., to deploy the artificial valve.

The distal unit may be disposable. It may be contained in a sterilized container.

The system may further comprise a positioning unit, in particular a robot, for positioning the common frame. This provides a simple and accurate positioning.

Moreover, the system may comprise a centering means for centering a catheter of the delivery system with respect to a secondary known axis, in particular that of a guidewire.

The system may also contain means for loading a guidewire into the delivery system. The means for loading may contain at least one of the following:
- a gripper with three translational degrees of freedom;
- a gripper with two translational degrees of freedom and one rotational degree of freedom, which may be implemented, for example, with at least one roller;
- centering means, for example a concentric gripper, and one translational degree of freedom.

The two translational degrees of freedom allow a centering.

The system may further comprise means for aligning the delivery system with respect to an introducer sheath which may be already present. The alignment with an introducer sheath may be achieved by a concentric gripper.

Moreover, the system may contain means for advancing the delivery system through the introducer sheath. Advancement of the delivery system through the introducer sheath may be achieved in one translational degree of freedom, for example with the aid of driving rollers.

In addition, the system may contain means for enabling valve deployment by actuation of the handle. Advantageously, these means are localized at the mechanical interface. A valve deployment through activation of the handle may contain one or two knob rotations. A friction wheel and a motor may be used for each knob. Alternatively, balloon expanding artificial valves exist which are partly or fully deployed by inflating a balloon (injecting pressurized fluid into a port situated on the handle). In this case, actuation triggers an inflation mechanism. Alternatively or in addition, the means for enabling valve deployment may contain a further actuation pack and mechanical interface to actuate an off-the-shelf inflation device.

Optionally, the system may comprise means for controlling and monitoring tool manipulations or navigation. For example, slippage, gripping and pushing/pulling forces, and/or tool tip localization may be controlled and/or monitored.

The invention further pertains to a cardiovascular system comprising a cardiovascular navigation and actuation system as described above, a positioning unit, in particular a robot, and a control unit for controlling the cardiovascular navigation and actuation system and the positioning unit. With such a system, the advantages explained above can be achieved in an automated manner.

The invention also pertains to a mechanical interface formed as one or more stand-alone adaptors. The adaptor contains a handle interface for connection with a handle of a cardiovascular delivery system. Moreover, it contains an actuation module interface for connection with an actuation module that is adapted for actuating the handle. This mechanical interface functions as an adaptor between the actuation module and an off-the-shelf handle. One specific adaptor may be provided for each type of handle. The mechanical interface allows to obtain a cardiovascular navigation and actuation system providing the advantages explained above. Moreover, the actuation module may be reused several times, even when the adaptor must be replaced for hygienic reasons.

Furthermore, the invention pertains to a kit of parts comprising
- a first adaptor with a first handle interface for connection with a first type of handle of cardiovascular delivery system,
- a second adaptor with a second handle interface for connection with a second type of handle of a cardiovascular delivery system.

The first type of handle is different from the second type of handle, but both the first adaptor and the second adaptor have a respective actuation module interface for creating an operative connection with one and the same type of actuation module.

This kit of parts has the advantage that one and the same actuation module interface may be used with handles of two or more delivery systems.

The adaptors may be disposable. They may be contained in a common sterilized container, or each component may be contained in a separate sterilized container.

The invention also pertains to a method of using a cardiovascular navigation and actuation system, in particular a cardiovascular navigation and actuation system as described above. This method comprises at least one of the following steps under the control of at least one automated actuator, in particular in the following order:
a. optionally, loading a proximal end of a guidewire into the tip of a delivery system;
b. optionally, advancing a delivery system coaxially on a guidewire to an access site, in particular advancing the delivery system of step a. coaxially on the guidewire of step a. to an access site;
c. optionally, aligning a delivery system with an introducer sheath that is positioned at an access site, in particular aligning the delivery system of any of the previous steps with an introducer sheath that is positioned at the access site of step b.;
d. optionally, pushing a tip of a delivery system through an introducer sheath, in particular pushing the tip of a delivery system of any of the previous steps through the introducer sheath of step c.;
e. advancing a delivery system, in particular the delivery system of any of the previous steps, to a cardiovascular site with a distal unit;
f. optionally, moving a positioning unit, in particular a robot, to a proximal part of the delivery system while maintaining an insertion position;
g. optionally, connecting a proximal unit having an actuation module to a handle of the delivery system via a mechanical interface;
h. positioning an artificial valve under the control of an actuation module, in particular the actuation module of step g.;
i. deploying an artificial valve, in particular an artificial valve of step h., under the control of an actuation module, in particular the actuation module of step g.;
j. optionally, validating proper positioning of the artificial valve;
k. optionally, moving a positioning unit, in particular the positioning unit of any of steps f. to j., to an access site, in particular the access site of any of steps b. to j.;
l. removing the delivery system (3).

The heart cycle of the patient may be monitored, and the motion of the delivery system may be synchronized by the control unit accordingly, in particular during passage of the artificial valve through the native valve. In particular, the distal actuation unit may be actuated only and thus the delivery system may only be advanced when the native valve is open.

The invention will be further explained below with reference to the attached drawings in which
- Figure 1: shows a first embodiment of a cardiovascular system according to the invention;
- Figure 2: shows a schematic second embodiment of a cardiovascular system according to the invention;
- Figure 3: shows a third embodiment of a cardiovascular system according to the invention;
- Figure 4: shows a fourth embodiment of a cardiovascular system according to the invention;
- Figure 5: shows a proximal unit of a schematic fifth embodiment of a cardiovascular system according to the invention;
- Figure 6: shows a proximal unit of a sixth embodiment of a cardiovascular system according to the invention;
- Figure 7: shows a distal unit of the sixth embodiment in a perspective view;
- Figure 8: shows the distal unit of Figure 7 in a top view;
- Figure 9: shows a distal unit of a seventh embodiment of a cardiovascular system according to the invention in a perspective view;
- Figure 10: shows the distal unit of Figure 9 in a top view;
- Figure 11: shows the sixth embodiment of a cardiovascular system in a perspective view;
- Figure 12: shows a seventh schematic embodiment of a cardiovascular system according to the invention;
- Figure 13: shows an introducer sheath fixation of an eighth embodiment of a cardiovascular system according to the invention;
- Figure 14: shows a guidewire driver of a ninth embodiment of a cardiovascular system according to the invention in a perspective view;
- Figure 15: shows the guidewire driver of Figure 14 in a side view;
- Figure 16: shows a portion of the ninth embodiment comprising a proximal unit and the guidewire driver of Figures 14 and 15;
- Figure 17: shows the ninth embodiment in a perspective view.

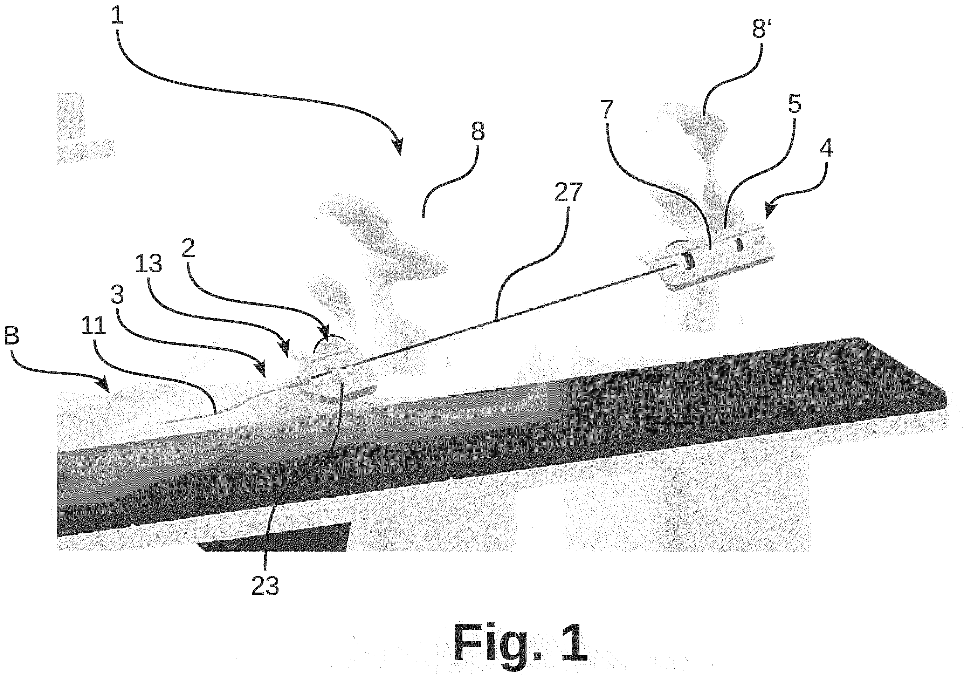

The cardiovascular navigation and actuation system 1 shown in Figure 1 contains a distal unit 2 which is held and positioned by a first robot 8 and a proximal unit 4 which is held and positioned by a second robot 8'. Thereby, the distal unit 2 and the proximal unit 4 can move independently from one another, which increases the range of applicability. Alternatively, the distal unit 2 and/or the proximal unit 4 may be held and positioned by passive positioning units or human operators.

The distal unit 2 is arranged near a percutaneous access site 13 at which an introducer sheath 11 is insertable into a patient's body B through the skin. It is configured for translating and/or rotating a cardiovascular delivery system 3, in particular a TAVI or a TAVR delivery system 3, along its longitudinal axis with the help of rollers 23.

The proximal unit 4 comprises a mechanical interface 5 and an actuation module 6 for actuating a handle 7 of the delivery system 3. The proximal unit 4 can manipulate and move the delivery system 3, in particular to deploy an artificial valve (not shown). The mechanical interface 5 provides a connection between the handle 7 and the actuation module (not shown). The catheter body 27 of the delivery system 3 slides concentrically along a guidewire (not shown) which has been placed in a left ventricle (i.e. the cardiovascular site) prior to this stage of the intervention.

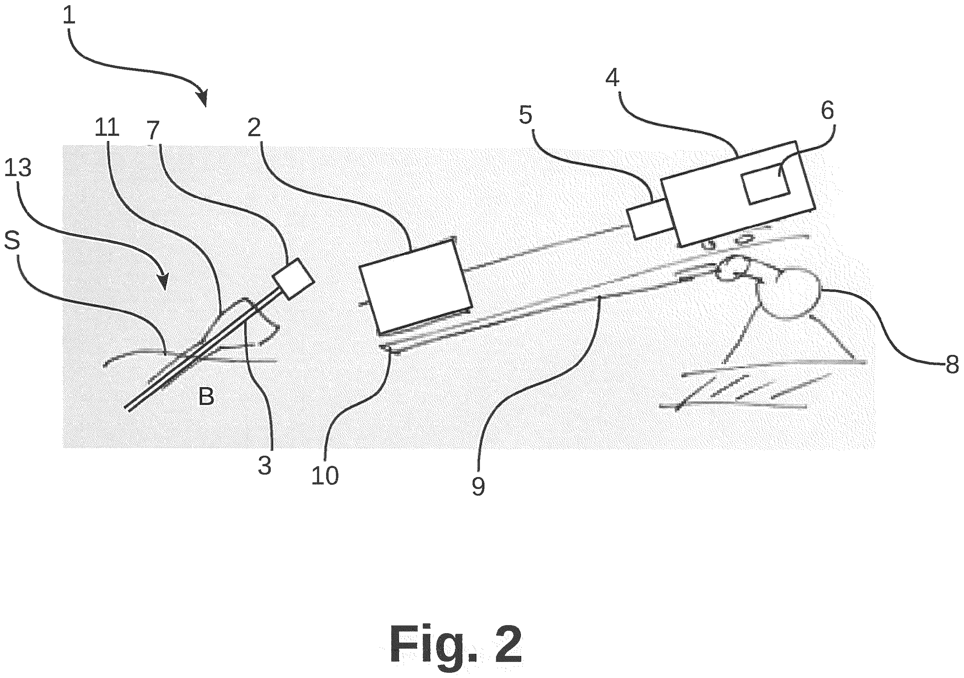

Figure 2 depicts another embodiment in which the distal unit 2 and the proximal unit 4 are mounted in a common frame 9 which is positioned by a robot 8. The proximal unit 4 is configured to be moved with respect to the distal unit 2 along the frame 9 and does so in function of the insertion need as dictated by the distal unit 2. This also allows an independent motion of these units and increases the range of applicability.

The distal unit 2 is fixed at a distal end 10 of the frame 9, which is in turn aligned with the percutaneous access site 13 at the patient. Once aligned, the distal unit 2 is engaged with the introducer sheath 11 at the access site 13 and can introduce and advance the delivery system 3.

The frame 9 may be first placed at the percutaneous access site 13 to insert the delivery system 3 in the patient and navigate it to the cardiovascular site. Doing so, it may pull the delivery system 3 which passively follows in the guiding means. By doing so, active control between the distal unit 2 and the proximal unit 4 can be avoided, thereby reducing the risk of a positional mismatch between units and unforeseen forces on the inserted instrumentation.

During insertion or after full insertion, the proximal unit 4 enables actuation of the handle 7 - for instance, positioning the valve seating along a limited longitudinal range and enabling valve expansion by balloon inflation or unsheathing a self-expanding valve.

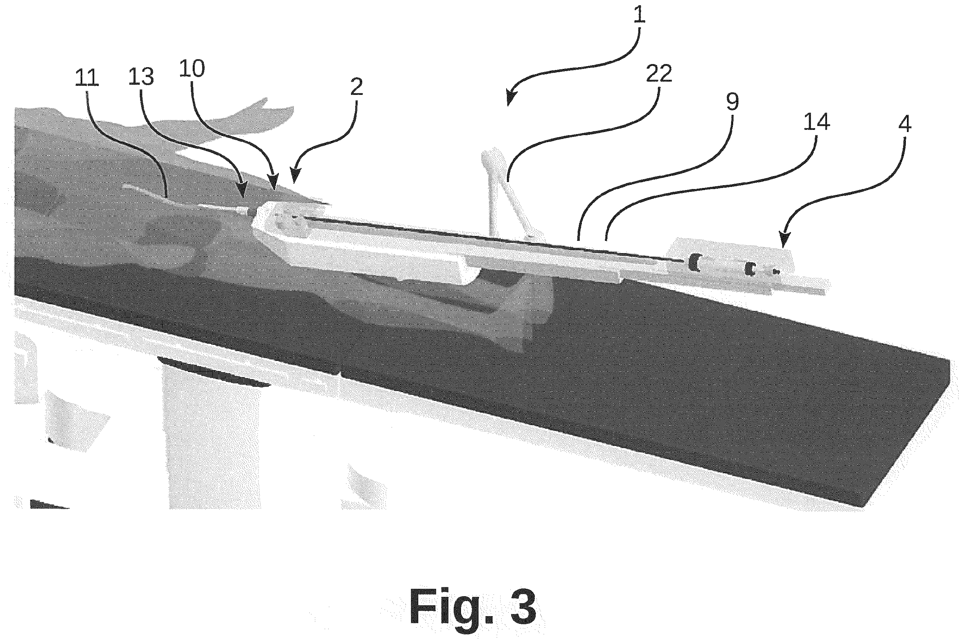

In the embodiment shown in Figure 3, the delivery system 3 is arranged on a linear guiderail 14. The distal unit 2 and the proximal unit 4 are mounted in a common frame 9 which is held by a passive telescopic positioning arm 22. In operation, the frame 9 is first placed at the percutaneous access site 13 to insert the delivery system 3 in the patient and navigate it to the cardiovascular site (not shown). Doing so, it pulls the delivery system 3 which passively follows in the guiderail 14. In this way, active control between the distal unit 2 and the proximal unit 4 can be avoided, thereby reducing the risk of a positional mismatch between the distal unit 2 and the proximal unit 4 and unforeseen forces on the inserted instrumentation. The frame 9 is then moved to the delivery system handle 7 to actuate the valve deployment.

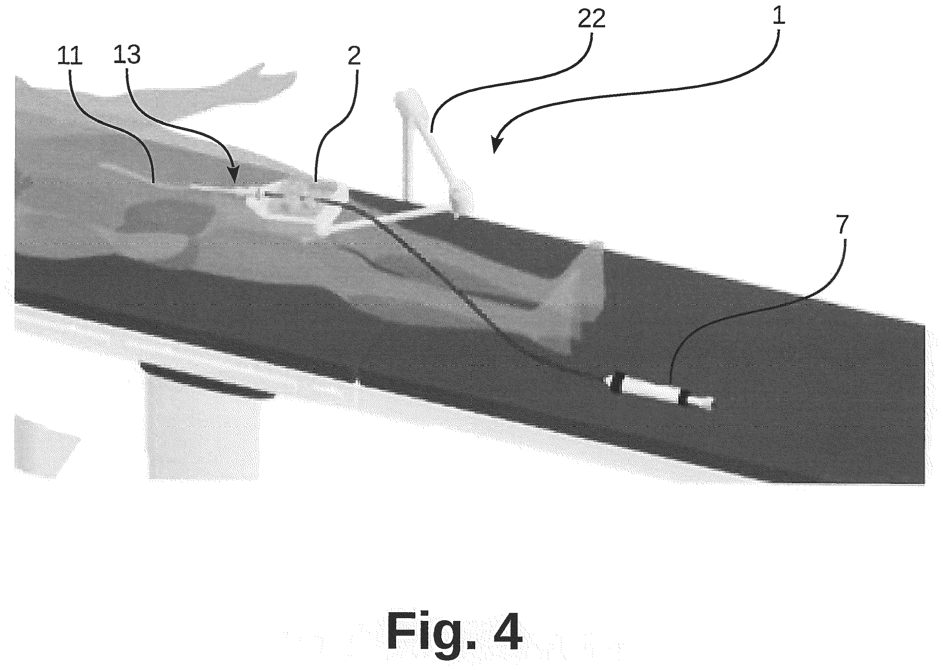

Figure 4 provides an embodiment which only contains a proximal unit 4, but not also a distal unit 2. Therefore, only one operator is needed.

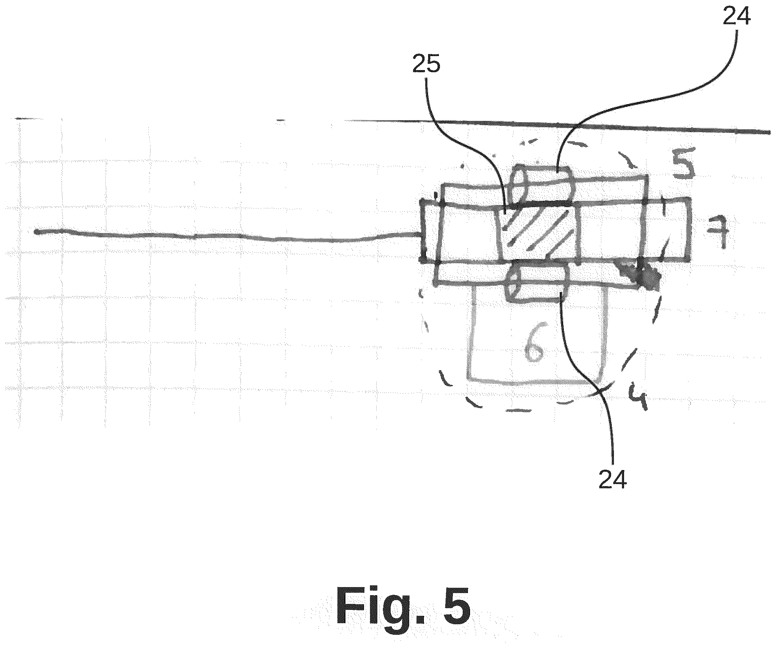

The embodiment shown schematically in Figure 5 comprises a proximal unit 4, a mechanical interface 5, and an actuation module 6. The proximal unit 4 is used for actuating a handle 7 of a delivery system. For this purpose, the proximal unit 4 contains gear wheels 24 which interact with a gear wheel 25 of the handle 7. Alternatively, friction wheels may be used instead of gear wheels.

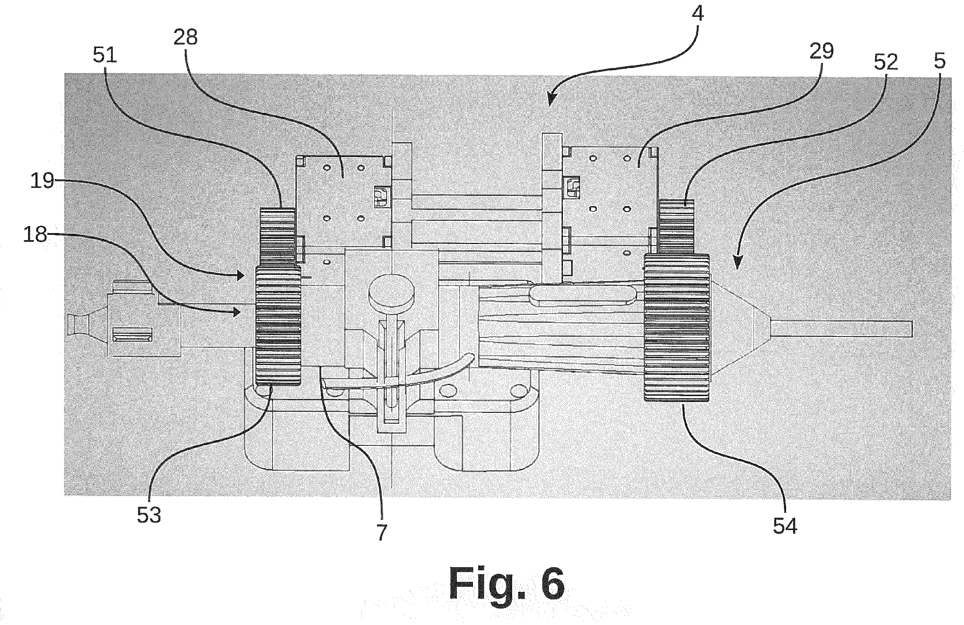

Figure 6 displays a proximal unit 4 comprising an actuation module 6 and a mechanical interface 5. The actuation module 6 contains a distal gear wheel 51 that can be actuated by a distal motor 28 and a proximal gear wheel 52 that can be actuated by a proximal motor 29. The mechanical interface 5 forms an adaptor between the actuation module 6 and the handle 7. It contains an actuation module interface 19 and a handle interface 18. The actuation module interface 19 comprises a distal gear wheel 53 which interacts with the distal gear wheel 51 of the actuation module 6 and a proximal gear wheel 54 which interacts with the proximal gear wheel 52 of the actuation module 6. The handle interface 18 couples the distal gear wheel 53 with a fine adjustment wheel (not shown) of the handle 7. In addition, the handle interface 18 couples the proximal gear wheel 54 with a flex wheel (not shown) of the handle 7.

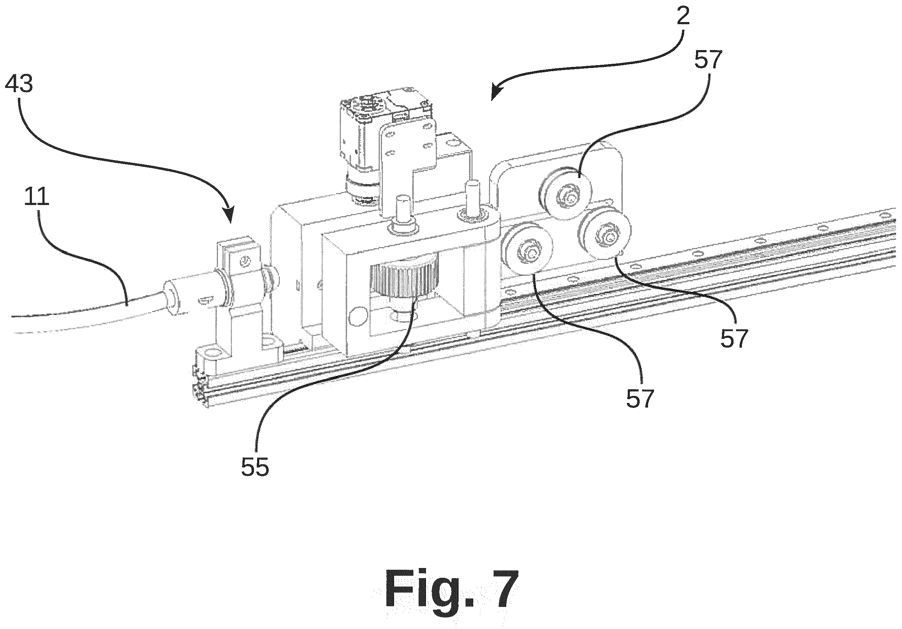

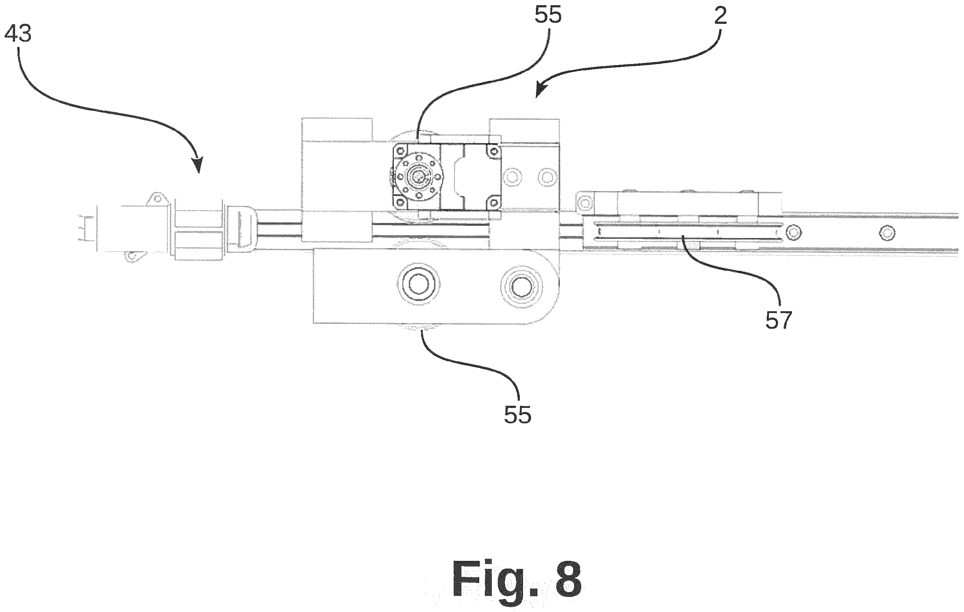

Figures 7 and 8 show, in two different perspectives, a distal unit 2 having opposing driving rollers 55 for advancing and/or retracting a catheter body (not shown), guiding rollers 57 for guiding the catheter body, and an introducer sheath fixation 43 for fixing the introducer sheath 11. In this embodiment, the rotation axes of the driving rollers 55 are arranged perpendicular to the rotation axes of the guiding rollers 57.

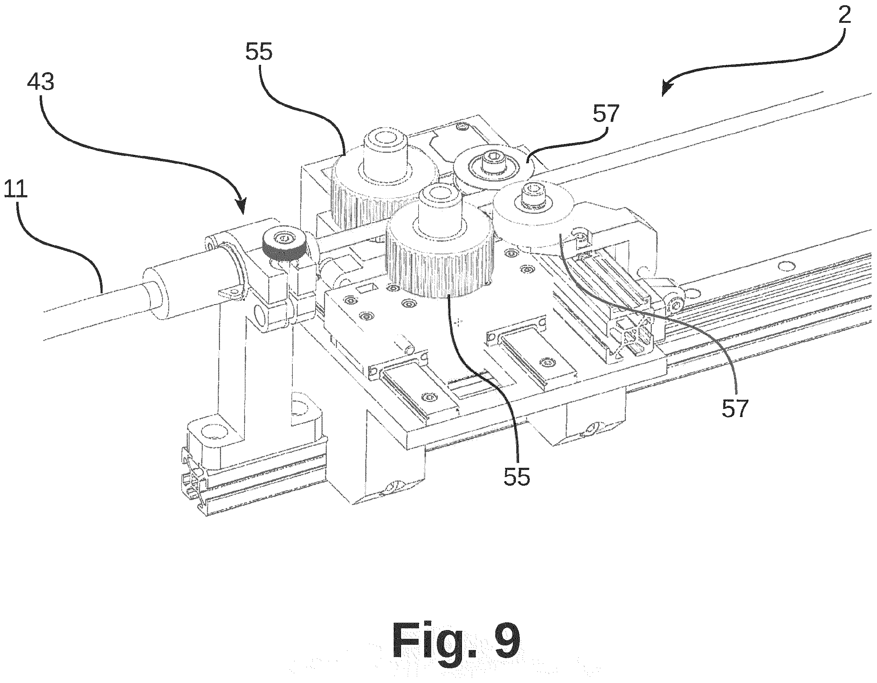

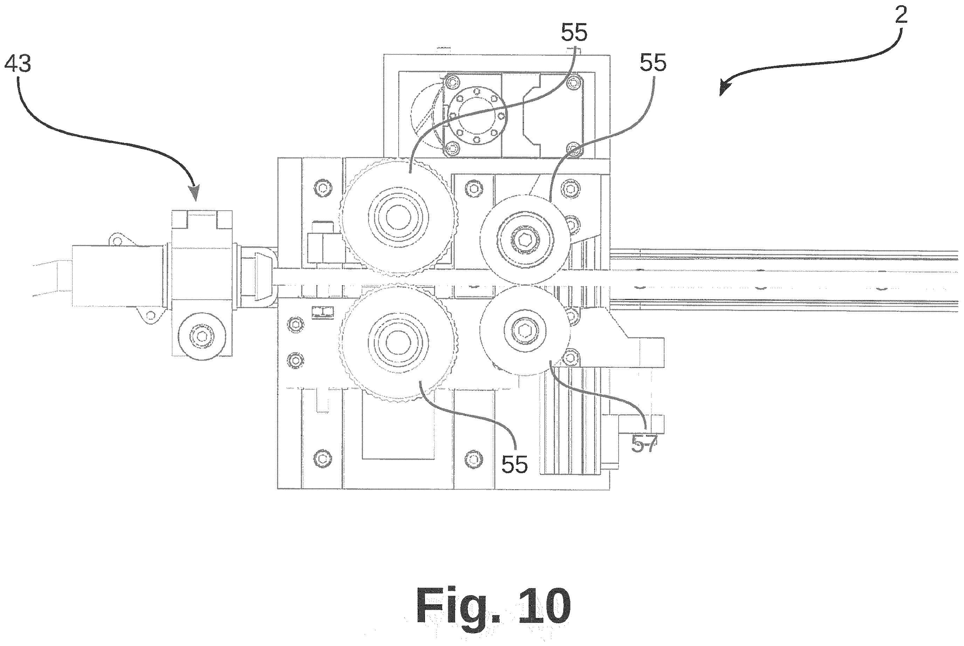

Figures 9 and 10 show, in two different perspectives, another distal unit 2 having opposing driving rollers 55 for advancing and/or retracting a catheter body (not shown), guiding rollers 57 for guiding the catheter body, and an introducer sheath fixation 43 for fixing the introducer sheath 11. Contrary to the embodiment shown in Figures 7 and 8, the rotation axes of the driving rollers 55 are arranged parallel to the rotation axes of the guiding rollers 57.

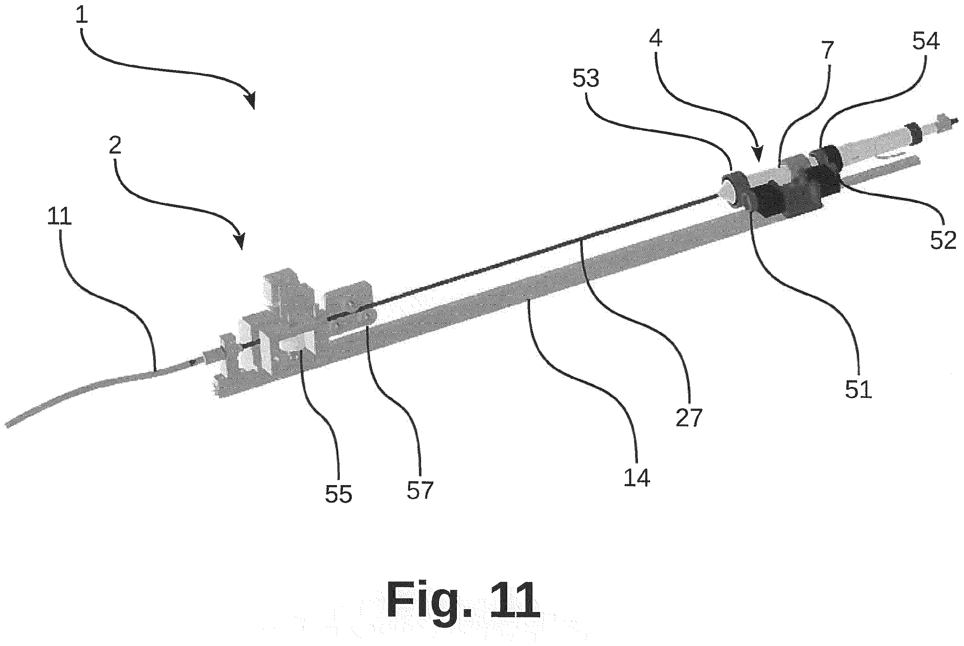

The embodiment shown in Figure 11 also comprises a distal unit 2 and a proximal unit 4. The distal unit 2 comprises a pair of opposing driving rollers 55 for translating a catheter body 27 in or out of the patient as well as guiding rollers 57 for guiding the catheter body 27. The proximal unit 4 is essentially identical to the one shown in Figure 6. The delivery system 3 displayed in Figure 11 is arranged on a linear guiderail 14. The distal unit 2 and the proximal unit 4 are mounted in a common frame 9 which is positioned by a robot 8.

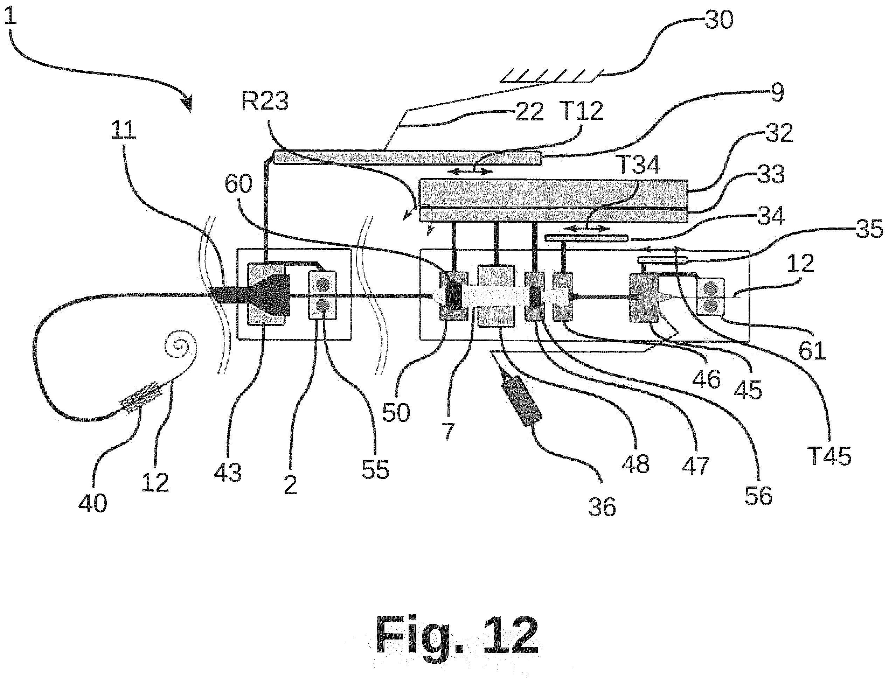

Figure 12 shows a further embodiment for positioning and delivering a balloon expandable valve with the aid of the Edwards Commander delivery system. The delivery system 3 comprises a catheter body 27, a guidewire 12, a balloon catheter including a balloon (not shown), an artificial valve 40, and a handle 7. The handle 7 comprises a flex wheel 60 for bending a tip of the catheter body 27, a fine adjustment wheel 56 for translating the balloon catheter inside the catheter body 27, and a balloon lock knob (not shown) for enabling or disabling the use of the fine adjustment wheel 56.

The cardiovascular navigation and actuation system 1 contains
- a reference frame 30, e.g., a side-rail of an operating room table,
- an introducer sheath 11 to be positioned and fixed at an access site of a patient;
- an introducer sheath fixation 43 for holding the introducer sheath 11, wherein the introducer sheath fixation 43 may comprise a rigid housing,
- a common frame 9 which is movable with respect to the reference frame 30, for holding and positioning the introducer sheath 11 and further components of the delivery system 3 and the cardiovascular navigation and actuation system 1,
- a positioning arm 22 for moving and positioning the fixation frame 9 relative to the reference frame and the patient by translations and/or rotations, wherein the positioning arm 22 may have a range from the side-rail of the operating room table to a midline of the patient, wherein the positioning arm 22 may have three or more degrees of freedom, wherein the positioning arm 22 may be lockable, wherein the positioning arm 22 may comprise at least one of: a cable brake, a screw at each link, a braked arm, and a robot arm),
- an insertion frame 32 which is movable with respect to the fixation frame 9, for at least partially inserting and/or retracting the delivery system 3 through the patient's anatomy by translations T12, wherein the insertion frame 32 may include two aluminium extrusion profiles slidably arranged with respect to each other via a plastic part interface, wherein the length of the extrusion profiles may be adapted to the patient's anatomy, e.g., about 40 cm,
- an actuation frame 33 which is movable with respect to the insertion frame 32 by rotations R23 with respect to the insertion frame 32 about an axial axis of the delivery system 3,
- a balloon-lock frame 34 which is movable with respect to the actuation frame 33 by translations T34, wherein the motion may be passive in function of the actuation of the fine adjustment wheel 56, which translates the balloon lock knob, wherein the balloon-lock frame 34 may be movable, for example, by 45 mm,
- a balloon-catheter frame 35 which is movable with respect to the balloon-lock frame 34 by translations T45, for example by 45 mm),
- a distal unit 2 for translating the catheter body 27 into or out of the patient, which distal unit 2 comprises a pair of motorized opposing driving rollers 55,
- a balloon inflation means 36 for inflating the balloon and/or deploying the artificial valve 40,
- a balloon shaft translation means 45 for translating the balloon catheter with respect to the catheter body 27, wherein the range of the translation may be, for example, 45 mm, wherein the balloon shaft translation means 45 may contain passive guiding means by which the balloon-lock frame 34 and the balloon-catheter frame 35 may be locked with respect to each other,
- a balloon lock (dis)engagement means 46 for rotating the balloon lock knob for enabling or disabling the use of the fine adjustment wheel 56,
- a fine adjustment wheel actuation means 47 for rotating the fine adjustment wheel 56, thereby translating the balloon catheter inside the catheter body 27,
- a handle fixation means 48 for fixing or releasing the handle 7 relative to the actuation frame 33,
- a flex wheel actuation means 50 for rotating the flex wheel 60, thereby pulling a cable to bend a tip of the catheter.

The distal unit 2 may comprise two actuators: a first one for radially gripping and a second one for longitudinally moving.

A radial gripping force may be generated by a device converting e.g. a circumferential movement into a radial movement, e.g. comparable to a drill chuck.

A voice coil may be used for stepwise longitudinal movement. The movement can be generated by a stepwise action, i.e. by gripping the catheter shaft at a first position, actuating the voice coil and translate the gripped catheter by a length corresponding to the maximum stroke of the voice coil, keeping the catheter at this position, releasing the gripping, and retracting the voice coil, re-grasping, advancing the voice coil etc. sequentially. In some embodiments, two voice coils can be used alternatingly, such that when one resets (gripper released, voice coil resetting position) the other voice coil placed in series manipulates the catheter (gripper engaged, voice coil controlling translation). Instead of a voice coil, any other device such as e.g. a belt and a wheel may also be used.

Instead of the insertion frame 32, a linear guiding rail may be provided. The translations T12 may be actively driven. Alternatively, the delivery system 3 may passively follow the distal unit 2.

In some embodiments, the handle 7 may perform rotations R23 about its longitudinal axis, for example within a range of ±45°. These rotations R23 may be performed either actively using an actuator and a mechanical joint (as for example rotary ring, go-niometric stage, etc.) or passively, where the handle 7 is fixated in a passive joint allowing the handle 7 to rotate in function of anatomical forces (i.e. the catheter remains in a lowest torsional energy state throughout insertion through tortuous anatomy).

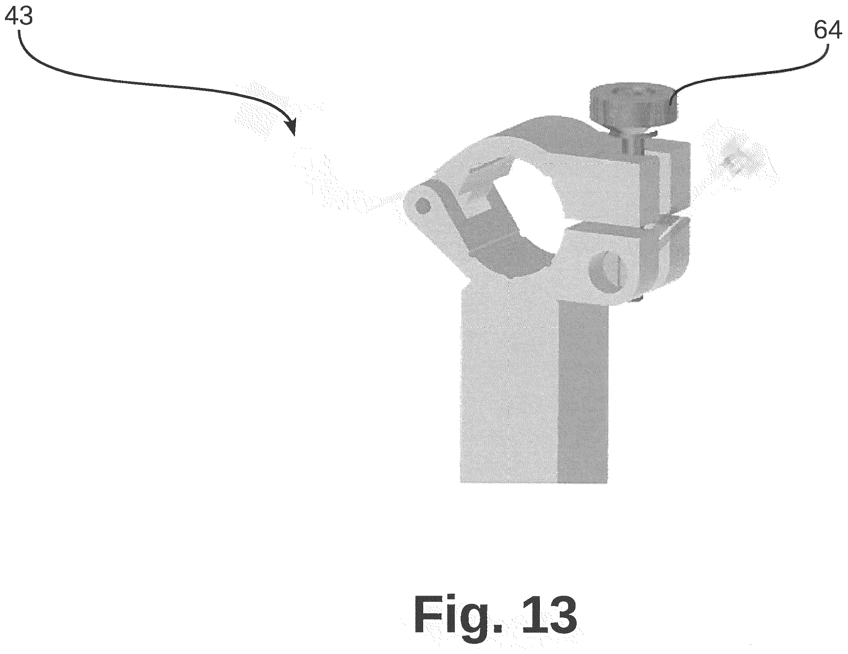

In Figure 13, a quick-release introducer sheath fixation 43 is shown which can be tightened and untightened by using a screw 64. Alternatively, a hinge and a thumbscrew may be used, allowing the introducer sheath fixation to hinge open for faster access.

The method according to the invention may replace two human operators. In more detail, the distal unit may perform the steps usually assigned to a first operator, and the proximal unit may perform the steps usually assigned to a second operator.

In a first step d., the delivery system 3 is introduced: The distal unit 2 fixates the introducer sheath 11, inserts the loaded catheter body 27 into the introducer sheath 11 and advances it until the tip of the delivery system 3 exits the distal end of the introducer sheath 11. The proximal unit 4 supports the handle 7 and pushes and/or pulls the guidewire 12 when necessary.

In a second step, the valve is aligned with respect to the balloon: The proximal unit 4 manipulates the balloon lock and the balloon catheter and pushes and/or pulls the guidewire 12 when necessary, while the distal unit 2 still fixates the introducer sheath 11.

In a third step e. of navigation, the distal unit 2 still fixates the introducer sheath 11 and advances the catheter body 27 through an aortic arch and then through a native valve, while the proximal unit 4 pushes the guidewire 12 and operates the flex wheel 60 of the handle 7.

In a fourth step of aligning the artificial valve 40 with the native anatomy, the proximal unit 4 operates the balloon inflation means 36 and pushes and/or pulls the guidewire 12 when necessary, while the distal unit 2 further fixates the introducer sheath 11 and pushes and/or pulls on the catheter body 27 when necessary.

In a fifth step h., the proximal unit 4 delivers the artificial valve 40 and operates the balloon inflation means 36, while the distal unit 2 still fixates the introducer sheath 11 and performs small adjustments of the catheter body 27 when necessary.

In a sixth step 1., the proximal unit 4 pulls the guidewire 12, while the distal unit 2 maintains the position of the introducer sheath 11 and retracts the catheter body 27.

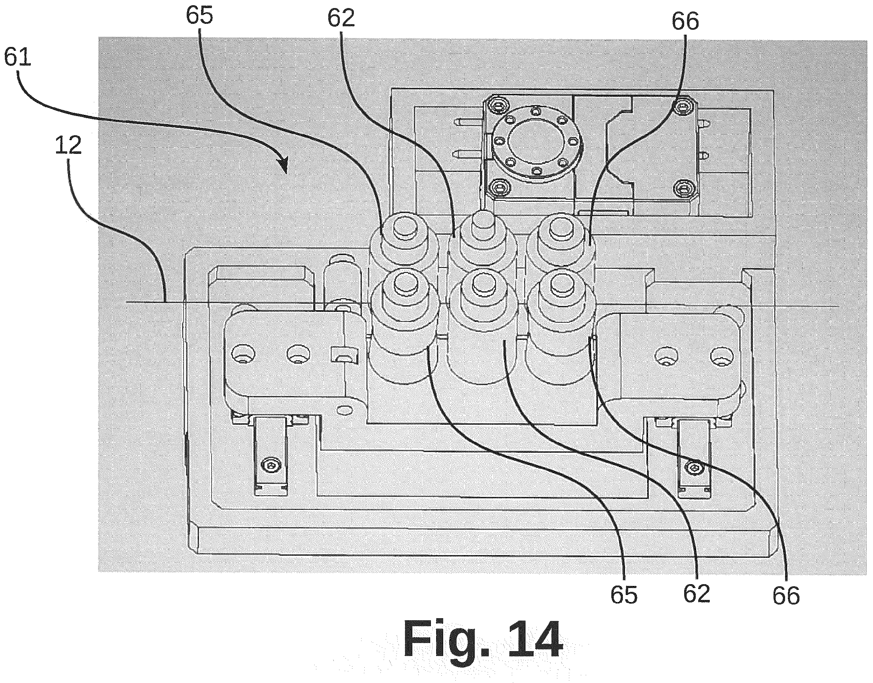

Figure 14 shows a guidewire driver 61 containing a pair of driving rollers 62 for driving the guidewire 12 and two pairs of guiding rollers 65, 66, wherein each pair contains a straight guiding roller 65 and a V-groove guiding roller 66. As seen in

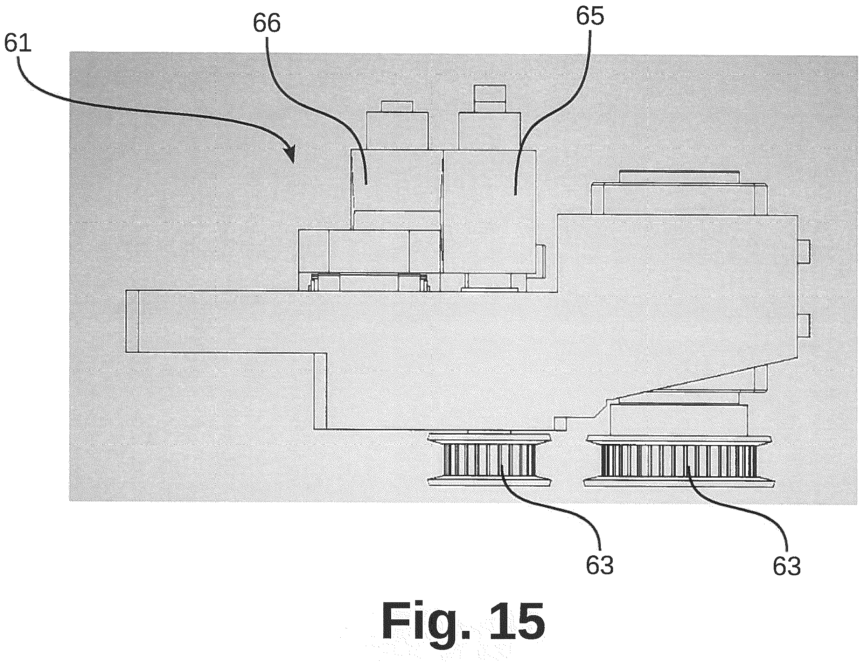

Figure 15, the guidewire driver 61 further contains two pulleys 63 for transferring forces from a motor and a timing belt (not shown) to the driving rollers 62.

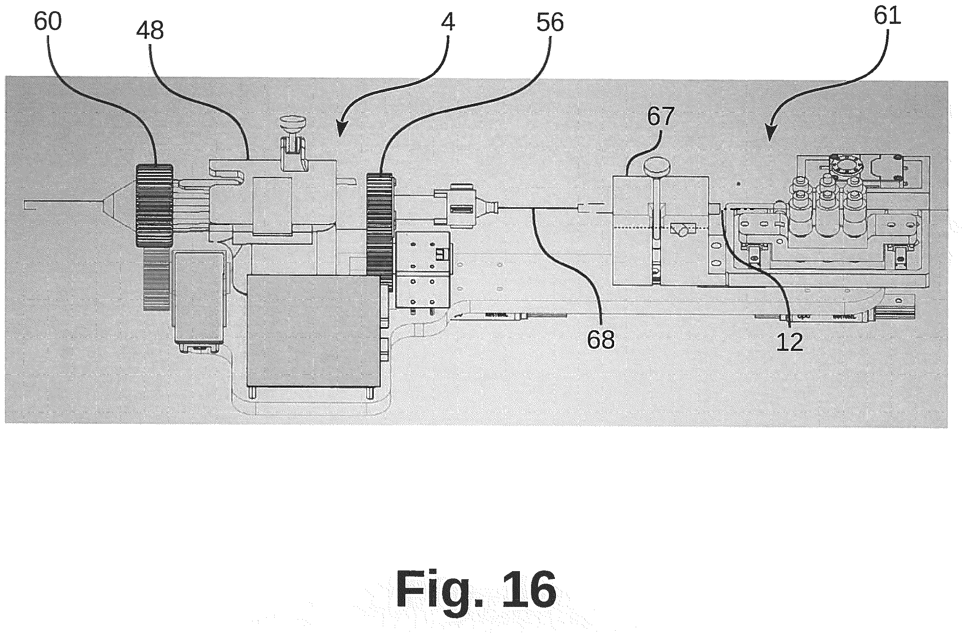

Figure 16 depicts a portion of a cardiovascular navigation and actuation system 1 for actuating a flex wheel 60, a handle fixation means 48, a fine adjustment wheel 56, and the guidewire driver 61 of Figures 12 and 13. Furthermore, a balloon Y connector fixation 67 and a balloon catheter shaft 68 are shown.

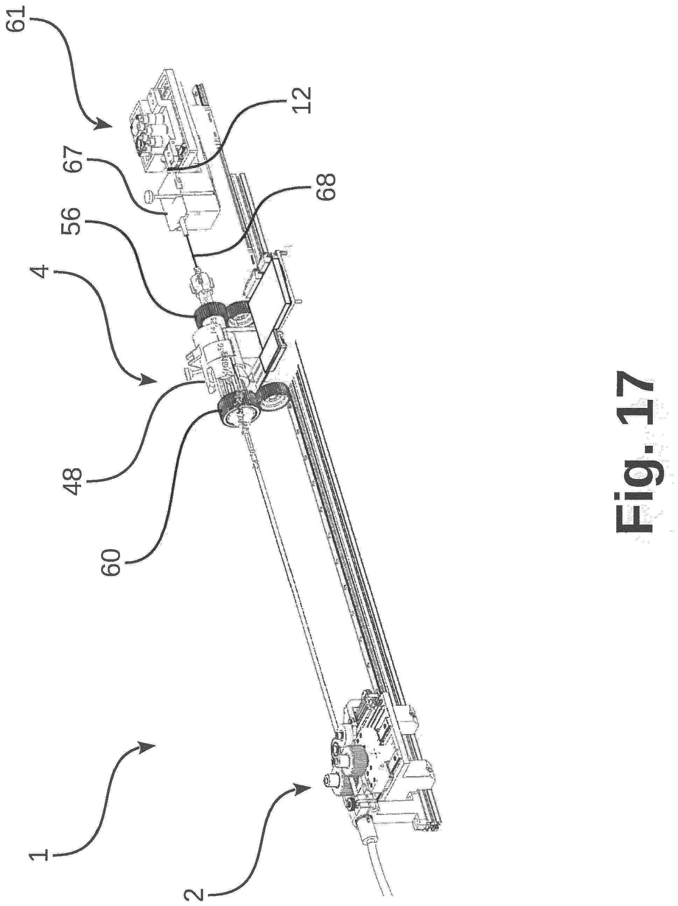

Figure 17 shows the cardiovascular navigation and actuation system 1 comprising the proximal unit 4 and the guidewire driver 1 of Figures 14 and 15 as well as a distal unit 2.

## Claims

1. A cardiovascular navigation and actuation system (1) comprising at least one of and preferably both of
- a distal unit (2) for translating and/or rotating a cardiovascular delivery system (3), in particular a TAVI or a TAVR delivery system, along its longitudinal axis;
- a proximal unit (4) comprising an actuation module (6) for actuating a function of a handle (7) of the delivery system (3), in particular to deploy an artificial cardiovascular valve (40).

2. The system (1) as claimed in claim 1,
wherein the proximal unit (4) further comprises a mechanical interface (5) which provides a connection between the handle (7) and the actuation module (6).

3. The system (1) as claimed in either of the preceding claims,
wherein the proximal unit (4) is mounted on a proximal positioning unit, in particular a proximal robot (8).

4. The system (1) as claimed in any of the preceding claims, wherein the distal unit (2) is mounted on a distal positioning unit, in particular a distal robot (8').

5. The system (1) as claimed in any of the preceding claims, wherein the proximal unit (4) and the distal unit (2) are arranged to move independently from one another.

6. The system (1) as claimed in any of the preceding claims, wherein the system (1) further comprises a common frame (9) in which the distal unit (2) and the proximal unit (4) are mounted.

7. The system (1) as claimed in claim 6,
wherein the distal unit (2) is fixed at a distal end (10) of the common frame (9).

8. The system (1) as claimed in one of claims 6 and 7,
wherein the system (1) further comprises a positioning unit, in particular a robot (8), for positioning the common frame (9).

9. The system (1) as claimed in any of the preceding claims, wherein the proximal unit (4) is movable with respect to the distal unit (2) and/or vice versa.

10. The system (1) as claimed in any of the preceding claims, wherein the proximal unit (4) is configured to be movable towards the distal unit (2) and to enable actuation of the delivery system (3).

11. The system (1) as claimed in any of the preceding claims, wherein the system comprises a centering means for centering a catheter with respect to a secondary known axis, in particular that of a guidewire (12).

12. Cardiovascular system comprising a cardiovascular navigation and actuation system (1) as claimed in any of the preceding claims, a positioning unit, in particular a robot (8), and a control unit for controlling the cardiovascular navigation and actuation system (1) and the positioning unit.

13. A mechanical interface (5) for providing a connection between a handle (7) of a cardiovascular delivery system (3) and an actuation module interface (19) for actuating the handle (7), wherein the mechanical interface (5) is formed as one or more stand-alone adaptors, each adaptor comprising
- a handle interface (18) for connection with the handle (7) and
- an actuation module interface (19) for connection with the actuation module (6).

14. A kit of parts comprising
- a first adaptor with a first handle interface (18) for connection with a first type of handle (7) of a cardiovascular delivery system (3) and
- a second adaptor with a second handle interface (18) for connection with a second type of handle (7) of a cardiovascular delivery system (3),
wherein
- the first type of handle (7) is different from the second type of handle (7) and
- both the first adaptor and the second adaptor have a respective actuation module interface (19) for creating an operative connection with one and the same type of actuation module (6).

15. Method of using a cardiovascular navigation and actuation system (1), in particular a cardiovascular navigation and actuation system (1) as described above, wherein the method comprises at least one of the following steps under the control of at least one automated actuator, in particular in the following order:
a. optionally, loading a proximal end of a guidewire (12) into the tip of a delivery system (3);
b. optionally, advancing a delivery system (3) coaxially on a guidewire (12) to an access site (13), in particular advancing the delivery system (3) of step a. coaxially on the guidewire (12) of step a. to an access site (13);
c. optionally, aligning a delivery system (3) with an introducer sheath (11) that is positioned at an access site (13), in particular aligning the delivery system (3) of any of the previous steps with an introducer sheath (11) that is positioned at the access site (13) of step b.;
d. optionally, pushing a tip of a delivery system (3) through an introducer sheath (11), in particular pushing the tip of a delivery system (3) of any of the previous steps through the introducer sheath (11) of step c.;
e. advancing a delivery system (3), in particular the delivery system (3) of any of the previous steps, to a cardiovascular site with a distal unit (2);
f. optionally, moving a positioning unit, in particular a robot (8), to a proximal part of the delivery system (3) while maintaining an insertion position;
g. optionally, connecting a proximal unit (4) having an actuation module (6) to a handle (7) of the delivery system (3) via a mechanical interface (5);
h. positioning an artificial valve (40) under the control of an actuation module (6), in particular the actuation module (6) of step g.;
i. deploying an artificial valve (40), in particular the artificial valve (40) of step h), under the control of an actuation module (6), in particular the actuation module (6) of step g;
j. optionally, validating proper positioning of the artificial valve (40);
k. optionally, moving a positioning unit, in particular the positioning unit of any of steps f. to j., to an access site (13), in particular the access site (13) of any of steps b. to i.;
l. removing the delivery system (3).
